# EUROPEAN PATENT APPLICATION

(11) **EP 3 981 411 A1**
(43) Date of publication of application: **13.04.2022**
(21) Application number: 21797288.4
(22) Date of filing: 25.03.2021
(51) Int. Cl.: A61K 31/57, A61K 39/395, A61P 31/00, A61P 31/06, A61P 31/12

(54) **APPLICATION OF PROGESTIN IN PREPARATION OF DRUG INHIBITING CYTOKINE STORM**

(30) Priority: 28.04.2020 CN 202010350632
(71) Applicant: Shenzhen Evergreen Therapeutics Co., Ltd., Shenzhen, Guangdong 518038 (CN)
(72) Inventor: DU, Tao Tom, Shenzhen, Guangdong 518038 (CN); DU, Xin, Shenzhen, Guangdong 518038 (CN)
(74) Representative: J A Kemp LLP
(86) International application number: PCT/CN2021/082899
(87) International publication number: WO 2021/218506

(57) **Abstract**

The present application provides a use of hydroxyprogesterone caproate for treating multiple diseases that cause a cytokine storm, such as novel coronavirus pneumonia, various virulent virus infections, side effects after monoclonal antibody treatment, side effects after CAR-T treatment, inflammatory bowel disease, and acute pancreatitis. Moreover, the present application also provides a method for treating the diseases by using a corresponding pharmaceutical composition. Experimental results show that progestin hydroxyprogesterone caproate can effectively inhibit the cytokine storm and is expected to be an effective drug for treating diseases such as novel coronavirus pneumonia.

## Description

### TECHNICAL FIELD

The disclosure belongs to the field of immune disease and infectious disease treatment, and in particular, this application provides use of PR2005 in the manufacture of a medicament for inhibiting a cytokine storm, especially a cytokine storm caused by diseases such as novel coronavirus pneumonia.

### BACKGROUD

Immune cells communicate with each other via cytokines. Cytokines are small molecules released by cells into the blood that can allow immune cells to move across the site of infection, phagocytose damaged cells, and even penetrate the walls of blood vessels; at the same time, cytokines can also trigger inflammation, causing swelling, fever and pain in the damaged body.

When the immune system is activated to a limited extent or out of control due to factors such as infection, CART treatment, certain drugs, or an imbalance in autoimmune regulation, a large variety of cytokines are released, and the level of a large number of pro-inflammatory cytokines rises sharply, which is called a cytokine storm syndrome (CSR). The cytokine storm syndrome is a systemic inflammatory reaction caused by hypersensitive activation of the immune system. Known specific symptoms include fever, headache, rash, arthralgia, myalgia, hypotension, vascular leakage, disseminated intravascular coagulation and even multiple organ failure, and in addition to cytokine levels, relevant indicators also include lymphopenia, elevated creatinine, disturbances in coagulation parameters, elevated ferritin and C protein, and the like. Clinically common causes of the cytokine storm include CART treatment, H5N1, H1N1, SARS, MERS, COVID-19, etc., and cytokines involved include mainly TNF-α, IL-1, IL-2, IL-6, IL-12, IFN-α, IFN-β, IFN-γ, MCP-1, IL-8, G-CSF, MCP-1, etc.

At present, the specific pathophysiological mechanism of the cytokine storm syndrome is unclear (presumably related to cytolytic immune response), and there is no special corresponding therapeutic drug. At present, most experience in clinical treatment of the cytokine storm syndrome comes from CRS symptoms caused by immunotherapy, especially adoptive cell therapy. In influenza virus and coronavirus infections, there are no clear treatment guidelines and drugs that have been attempted to treat the cytokine storm syndrome include peroxisome proliferator-activated receptor agonists, sphingosine-1-phosphate receptor agonists, cyclooxygenase inhibitors, antioxidants, anti-tumor necrosis factor therapy, intravenous immunoglobulin and other therapies.

Novel coronavirus pneumonia (COVID-19), caused by novel coronavirus SARS-CoV-2, is a major threat to human health and social and economic development. A significant proportion of deaths due to COVID-19 are associated with the cytokine storm (Mehta P et al., COVID-19: consider cytokine storm syndromes and immunosuppression, Lancet, Vol. 395, published online on March 16, 2020; Fu B et al., Pathogenic T cells and inflammatory monocytes incite inflammatory storm in severe COVID-19 patients, J Transl Med, April 2004, Vol. 18, No. 1). Inhibition of the cytokine storm syndrome, like inhibition of viral invasion/replication, is one of the main ways to treat COVID-19 and reduce the mortality of COVID-19.

### SUMMARY

In order to inhibit a cytokine storm and treat a series of diseases associated therewith, we screened a large number of candidate drugs, among which hydroxyprogesterone caproate in the research and development stage is a candidate drug with the potential of high efficacy and low side effects. The test results show that hydroxyprogesterone caproate can inhibit the cytokine storm in an animal model. Our results show that PR2005 has the potential to be a clinical drug for the treatment of COVID-19.

In one aspect, the present application seeks to protect use of progestogen in the manufacture of a medicament for inhibiting a cytokine storm syndrome.

In another aspect, the present application seeks to protect a pharmaceutical composition for inhibiting a cytokine storm syndrome, including progestogen.

In yet another aspect, the present application seeks to protect a method for treating a coronavirus-infected disease, including administering progestogen.

The progestogen may be selected from hydroxyprogesterone caproate, medroxyprogesterone acetate, progesterone and the like, preferably hydroxyprogesterone caproate.

The cytokine storm syndrome may be induced by various virus infections, macromolecular antibody therapy, organ transplantation or chimeric antigen receptor T cell (CAR-T) therapy.

The infection may be coronavirus infection, influenza virus infection, and other virus infection.

The coronavirus-infected disease may be novel coronavirus pneumonia, Middle East respiratory syndrome and severe acute respiratory syndrome, preferably novel coronavirus pneumonia, and these diseases are treated by administering the progestogen to inhibit the cytokine storm syndrome in these diseases.

The novel coronavirus described in the present application has a variety of nomenclature, including, but not limited to, novel coronavirus, 2019-nCov and SARS-CoV-2 (officially named by International Committee on Taxonomy of Viruses); the disease caused after infection with the virus may be referred to as novel coronavirus pneumonia, NCP, COVID-19 (officially named by the International Health Organization), and the like.

Hydroxyprogesterone caproate in the present application is also referred to as 17α-hydroxyprogesterone caproate, 17-HPC, 17-hydroxyprogesterone caproate, 17-hydroxyhexylprogesterone or PR2005 with a CAS number of 630-56-8 used in the experimental part.

The medicament in the present application may be in any clinically acceptable dosage form and the medicament in the treatment of the present application may be administered in any clinically acceptable dosage form. Specific dosage forms include, but are not limited to, tablets, capsules, oral liquids, injections, powder injections, and the like.

The medicament prepared by the present disclosure may also contain other known and unknown drugs for the treatment of relevant diseases, other known and unknown drugs for the treatment of relevant diseases may also be used in the treatment method of the present disclosure, these drugs include, but are not limited to, drugs used to treat autoimmune diseases, such as immunosuppressants, peroxisome proliferator-activated receptor agonists, sphingosine-1-phosphate receptor agonists, cyclooxygenase inhibitors, antioxidants, anti-tumor necrosis factor therapy, intravenous immunoglobulin, and other therapies; drugs used to treat infections, such as antibiotics, antifungal drugs, viral replication inhibitors, and viral invasion inhibitors; and known and unknown drugs to treat symptoms such as fever, vomiting, skin problems in related diseases.

According to the dosage form prepared/administrated, the medicament may adopt various pharmaceutically acceptable excipients, including, but not limited to, coating materials, solvents, solubilizers, binders, stabilizers, antioxidants, pH regulators and flavoring agents, and these excipients may be selected by those skilled in the art in view of general knowledge of pharmacy.

### DETAILED DESCRIPTION

### Test animals and materials:

Humanized mice bred under specific pathogen-free conditions were used in the test. All test procedures were approved by the Animal Ethics Committee. All antibodies used for flow cytometry were purchased from outside. The endotoxin levels of these antibodies were all less than 0.5 IU/mg.

Human peripheral blood mononuclear cells (PBMCs) were also used in this test. Isolated human PBMCs were washed with phosphate buffer. After lysing red blood cells, the nucleated cells were washed for three times with phosphate buffer.

### Test method and procedures:

In general, the ratio of human CD45 cells to mouse CD45 cells in the spleen and blood of humanized severe combined immunodeficient (hu-SCID) mice was 3:1 and 0.4:1, respectively, after post-adaptive transfer of human peripheral blood mononuclear cells (PBMCs) for 10 days. This was equivalent to (1-2)×10⁷ human CD45 cells in the spleen and 1×10⁶ human CD45 cells per milliliter of blood.

It was reported that muromonab-CD3 (OKT3) will produce severe adverse effects after infusion. After 10 days of adoptive transfer of human PBMCs, human cells isolated from the spleen of the hu-SCID mice were still able to bind to OKT3.

In the test, we chose both intravenous (IV) and intraperitoneal (IP) routes of administration because antibody absorption was slower after intraperitoneal injection.

Each humanized mouse for the test was first intravenously injected with 2×10⁷ PBMCs. After 10 days, each mouse was injected with OKT3 or control IgG (2 µg or 10 µg, IV or IP).

Blood was collected from the mice at 10 min, 20 min and 60 min after administration of the OKT3 and control IgG A multiplex immunoassay system was then applied to the collected heparinized plasma to analyze cytokines. Cytokines measured included human IL-1β, IL-2, IL-4, IL-5, IL-6, IL-10, IL-12 (p70), IL-13, IL-17, IFN-γ and TNF-α. The mice were sacrificed after last blood collection (60 min). The activation markers thereof were then analyzed by flow cytometry. Human peripheral blood mononuclear cells in the mouse spleen were blocked with 2.4G2 and then stained with fluorescently labeled CD69, CD25, CD45 and CD3 .

Body temperature measurement: the rectal temperature of mice was measured before treatment and the rectal temperature was measured again before each blood collection point. The temperature was measured by inserting a rectal thermocouple probe and waiting for the number to stabilize (about 10s) to obtain the reading. Mice with a body temperature of smaller than 32°C were sacrificed.

Clinical score measurement: as recommended by the Canadian Council for Animal Care (www.ccac.ca/en_/standards/guidelines), we conducted preliminary studies to determine the most appropriate clinical symptoms and scoring criteria. Mice were observed at each time point and given one clinical score. Score: 0 = normal activity; 1 = normal activity, hairs upright, and tiptoe gait; 2 = arched back, decreased activity, but there are still activities; 3 = too few activities, but there are still activities when prompted; 4 = dying. Mice with a clinical score of 4 were sacrificed. Statistical analysis was then performed on inter-group differences in the test data.

### Test results:

Symptom observation after injection of antibody:

### Animals injected with OKT3:

IP injection group: in the administration of the IP route at a low dose of 2µg, all hu-SCID mice were unaffected. Hu-SCID mice receiving IP administration of 10µg OKT3 were clinically scored as moderate (arched back, too few activities) and one of 5 mice was dying and sacrificed at 2h.

IV injection group: Hu-SCID mice showed moderate to severe response within 1h when intravenously given 2 µg of OKT3. Two of four mice needed to be euthanized, while the remaining two mice showed moderate symptoms but slowly recovered within 5 hours. The response of mice to 10 µg of OKT3 intravenously injected was severe, so all mice were sacrificed at 1h.

### Animals injected with polyclonal antibody ATG:

No clinical symptoms were found in the hu-SCID mice injected with control IgG by either IP or IV administration.

### Hypothermia after antibody injection

Both hypothermia and hyperthermia occur in the cytokine storm. To this end, we made observations.

OKT3 group: the rectal temperature decreased significantly from 37°C to 32°C or below within 1h, after IP or IV injection of 10 µg OKT3. At a lower dose of 2 µg, administration of the IV route resulted in transient hypothermia (about 32°C) at 1h with partial recovery after 5h; the moderate changes that occurred after administration of the IP route were similar to those that occurred after injection of control human IgG

IgG control group: no apparent change in body temperature.

Circulating cytokine changes after antibody injection:
After antibody injection, in order to determine if a cytokine storm was induced, we detected cytokines (human IL-1β, IL-2, IL-4, IL-5, IL-6, IL-10, IL-12 (p70), IL-13, IL17, IFN-γ and TNF-α) in the plasma of the hu-SCID mice.

OKT3 group: OKT3-IP intraperitoneally injected at a high dose (10 µg) may induce production of multiple cytokines, including IL-1β, IL-2, IL-4, IL-5, IL-6, IL-10 and IFN-γ, and concentrations thereof gradually increased within 5 h of the test; in addition, production of TNF-α was also induced, but its peak appeared earlier (at 1h). Hu-SCID mice that received IV injection of high-dose OKT3 were sacrificed after 1h, so cytokines after this time point could not be detected. Induced cytokines were rarely detected at any time point, regardless of whether 2 µg of OKT3 was injected intraperitoneally or intravenously.

IgG control group: no cytokine changes were observed.

PR2005 treatment group:
On days 8 and 9 after intravenous injection of PBMCs in humanized mice (48 hours and 24 hours before antibody injection), a candidate drug PR2005 (5 mg/kg) was injected intraperitoneally to the animals. After the use of PR2005, the animals showed no significant changes in body temperature after receiving OKT3. In the animal group using PR2005 and OKT3 simultaneously, the concentration of cytokines in the blood was also substantially lower than that of animals using OKT3 alone. The results are detailed in Tables 1-4.

**Table 1. Rectal temperature decline in hu-SCID mice induced by OKT3 injection. The data is the mean value of each group of animals. Compared with the control group at the same time point.**

| | IgG | | | | OKT3 | | | | | | | | |
|---|---|---|---|---|---|---|---|---|---|---|---|---|---|
| | 10µg | | | | 2µg | | | | 10µg | | | | |
| Time (hrs) | 0 | 1 | 2 | 5 | 0 | 1 | 2 | 5 | 0 | 1 | 2 | 5 | |
| Temp | 37.7 | 36.6 | 35.3 | 36.1 | 37.7 | 35.6 | 34.8 | 35.4 | 37.7 | 31.7 | 31.1 | 30.6 | IP |
| | 37.7 | 35.7 | 34.5 | 35.0 | 37.7 | 31.7 | 33.8 | 34.9 | 37.7 | 31.9 | | | IV |

**Table 2. Cytokines induced after OKT3 injection. Hu-SCID mice were intraperitoneally injected with 10 µg of OKT3 (black bars) or IgG control (grey bars). Blood samples were taken at 1h, 2h and 5h respectively to determine the concentration of circulating cytokines. The sensitivity (limit of detection) of the analytical method was 1 ng·ml⁻¹. The data is expressed as the mean value of each group of animals. Compared with the control group at the same time point.**

| | IL-1β | | | | | |
|---|---|---|---|---|---|---|
| Time (hrs) Concentration (pg/ml) | 1 | | 2 | | 5 | |
| | 30.0 | 44.0 | 30.0 | 77.5 | 30.0 | 97.4 |
| | IgG | OKT3 | IgG | OKT3 | IgG | OKT3 |

| | IL-4 | | | | | |
|---|---|---|---|---|---|---|
| Time (hrs) Concentration (pg/ml) | 1 | | 2 | | 5 | |
| | 33 | 61 | 40 | 182 | 37 | 259 |
| | IgG | OKT3 | IgG | OKT3 | IgG | OKT3 |

| | IL-6 | | | | | |
|---|---|---|---|---|---|---|
| Time (hrs) Concentration (pg/ml) | 1 | | 2 | | 5 | |
| | 35 | 96 | 35 | 160 | 35 | 252 |
| | IgG | OKT3 | IgG | OKT3 | IgG | OKT3 |

| | IFN-γ | | | | | |
|---|---|---|---|---|---|---|
| Time (hrs) Concentration (pg/ml) | 1 | | 2 | | 5 | |
| | 1910 | 7015 | 2119 | 23574 | 1597 | 25783 |
| | IgG | OKT3 | IgG | OKT3 | IgG | OKT3 |

| | IL-2 | | | | | |
|---|---|---|---|---|---|---|
| Time (hrs) Concentration (pg/ml) | 1 | | 2 | | 5 | |
| | 23.4 | 39.0 | 21.5 | 75.9 | 19.3 | 89.4 |
| | IgG | OKT3 | IgG | OKT3 | IgG | OKT3 |

| | IL-5 | | | | | |
|---|---|---|---|---|---|---|
| Time (hrs) Concentration (pg/ml) | 1 | | 2 | | 5 | |
| | 57 | 72 | 57 | 98 | 57 | 158 |
| | IgG | OKT3 | IgG | OKT3 | IgG | OKT3 |

| | IL-10 | | | | | |
|---|---|---|---|---|---|---|
| Time (hrs) Concentration (pg/ml) | 1 | | 2 | | 5 | |
| | 694 | 3319 | 694 | 11001 | 694 | 20052 |
| | IgG | OKT3 | IgG | OKT3 | IgG | OKT3 |

| | TNF-α | | | | | |
|---|---|---|---|---|---|---|
| Time (hrs) Concentration (pg/ml) | 1 | | 2 | | 5 | |
| | 51 | 1409 | 51 | 1127 | 51 | 655 |
| | IgG | OKT3 | IgG | OKT3 | IgG | OKT3 |

**Table 3. 48 hours prior to antibody injection, a candidate drug PR2005 (5 mg/kg) was intraperitoneally injected to animals. Blood samples were taken at 1h, 2h and 5h respectively to determine the concentration of circulating cytokines. The sensitivity (limit of detection) of the analytical method was 1 ng·ml⁻¹. The data is expressed as the mean value of each group of animals. Compared with the control group at the same time point.**

| | IL-1β | | | | | | | | |
|---|---|---|---|---|---|---|---|---|---|
| Time (hrs) Concentration (pg/ml) | 1 | | | 2 | | | 5 | | |
| | 30.0 | 37.4 | 44.0 | 30.0 | 69.1 | 77.5 | 30.0 | 93.5 | 97.4 |
| | IgG | PR2005+ OKT3 | OKT3 | IgG | PR2005+ OKT3 | OKT3 | IgG | PR2005+ OKT3 | OKT3 |

| | IL-4 | | | | | | | | |
|---|---|---|---|---|---|---|---|---|---|
| Time (hrs) Concentration (pg/ml) | 1 | | | 2 | | | 5 | | |
| | 33 | 49 | 61 | 40 | 151 | 182 | 37 | 221 | 259 |
| | IgG | PR2005+ OKT3 | OKT3 | IgG | PR2005+ OKT3 | OKT3 | IgG | PR2005+ OKT3 | OKT3 |

| | IL-6 | | | | | | | | |
|---|---|---|---|---|---|---|---|---|---|
| Time (hrs) Concentration (pg/ml) | 1 | | | 2 | | | 5 | | |
| | 35 | 88 | 96 | 35 | 123 | 160 | 35 | 146 | 252 |
| | IgG | PR2005+ OKT3 | OKT3 | IgG | PR2005+ OKT3 | OKT3 | IgG | PR2005+ OKT3 | OKT3 |

| | IFN-γ | | | | | | | | |
|---|---|---|---|---|---|---|---|---|---|
| Time (hrs) Concentration (pg/ml) | 1 | | | 2 | | | 5 | | |
| | 1910 | 5550 | 7015 | 2119 | 13231 | 23574 | 1597 | 18388 | 25783 |
| | IgG | PR2005+ OKT3 | OKT3 | IgG | PR2005+ OKT3 | OKT3 | IgG | PR2005+ OKT3 | OKT3 |

| | IL-2 | | | | | | | | |
|---|---|---|---|---|---|---|---|---|---|
| Time (hrs) Concentration (pg/ml) | 1 | | | 2 | | | 5 | | |
| | 23.4 | 32.7 | 39.0 | 21.5 | 65.7 | 75.9 | 19.3 | 70.4 | 89.4 |
| | IgG | PR2005+ | OKT3 | IgG | PR2005+ | OKT3 | IgG | PR2005+ | OKT3 |
| | | OKT3 | | | OKT3 | | | OKT3 | |

| | IL-5 | | | | | | | | |
|---|---|---|---|---|---|---|---|---|---|
| Time (hrs) Concentration (pg/ml) | 1 | | | 2 | | | 5 | | |
| | 57 | 71 | 72 | 57 | 81 | 98 | 57 | 115 | 158 |
| | IgG | PR2005+ OKT3 | OKT3 | IgG | PR2005+ OKT3 | OKT3 | IgG | PR2005+ OKT3 | OKT3 |

| | IL-10 | | | | | | | | |
|---|---|---|---|---|---|---|---|---|---|
| Time (hrs) Concentration (pg/ml) | 1 | | | 2 | | | 5 | | |
| | 694 | 3085 | 3319 | 694 | 6885 | 11001 | 694 | 15016 | 20052 |
| | IgG | PR2005+ OKT3 | OKT3 | IgG | PR2005+ OKT3 | OKT3 | IgG | PR2005+ OKT3 | OKT3 |

| | TNF-α | | | | | | | | |
|---|---|---|---|---|---|---|---|---|---|
| Time (hrs) Concentration (pg/ml) | 1 | | | 2 | | | 5 | | |
| | 51 | 1383 | 1409 | 51 | 733 | 1127 | 51 | 442 | 655 |
| | IgG | PR2005+ OKT3 | OKT3 | IgG | PR2005+ OKT3 | OKT3 | IgG | PR2005+ OKT3 | OKT3 |

**Table 4. 24 hours prior to antibody injection, a candidate drug PR2005 (5 mg/kg) was intraperitoneally injected to animals. Blood samples were taken at 1h, 2h and 5h respectively to determine the concentration of circulating cytokines. The sensitivity (limit of detection) of the analytical method was 1 ng·ml⁻¹. The data is expressed as the mean value of each group of animals. Compared with the control group at the same time point.**

| | IL-1β | | | | | | | | |
|---|---|---|---|---|---|---|---|---|---|
| Time (hrs) Concentration (pg/ml) | 1 | | | 2 | | | 5 | | |
| | 30.0 | 35.4 | 44.0 | 30.0 | 44.1 | 77.5 | 30.0 | 39.8 | 97.4 |
| | IgG | PR2005+ OKT3 | OKT3 | IgG | PR2005+ OKT3 | OKT3 | IgG | PR2005+ OKT3 | OKT3 |

| | IL-4 | | | | | | | | |
|---|---|---|---|---|---|---|---|---|---|
| Time (hrs) Concentration (pg/ml) | 1 | | | 2 | | | 5 | | |
| | 33 | 39 | 61 | 40 | 114 | 182 | 37 | 132 | 259 |
| | IgG | PR2005+ OKT3 | OKT3 | IgG | PR2005+ OKT3 | OKT3 | IgG | PR2005+ OKT3 | OKT3 |

| | IL-6 | | | | | | | | |
|---|---|---|---|---|---|---|---|---|---|
| Time (hrs) Concentration | 1 | | | 2 | | | 5 | | |
| | 35 | 51 | 96 | 35 | 70 | 160 | 35 | 69 | 252 |
| (pg/ml) | | | | | | | | | |
| | IgG | PR2005+ OKT3 | OKT3 | IgG | PR2005+ OKT3 | OKT3 | IgG | PR2005+ OKT3 | OKT3 |

| | IFN-γ | | | | | | | | |
|---|---|---|---|---|---|---|---|---|---|
| Time (hrs) Concentration (pg/ml) | 1 | | | 2 | | | 5 | | |
| | 1910 | 3960 | 7015 | 2119 | 4621 | 23574 | 1597 | 11359 | 25783 |
| | IgG | PR2005+ OKT3 | OKT3 | IgG | PR2005+ OKT3 | OKT3 | IgG | PR2005+ OKT3 | OKT3 |

| | IL-2 | | | | | | | | |
|---|---|---|---|---|---|---|---|---|---|
| Time (hrs) Concentration (pg/ml) | 1 | | | 2 | | | 5 | | |
| | 23.4 | 29.7 | 39.0 | 21.5 | 40.1 | 75.9 | 19.3 | 39.7 | 89.4 |
| | IgG | PR2005+ OKT3 | OKT3 | IgG | PR2005+ OKT3 | OKT3 | IgG | PR2005+ OKT3 | OKT3 |

| | IL-5 | | | | | | | | |
|---|---|---|---|---|---|---|---|---|---|
| Time (hrs) Concentration (pg/ml) | 1 | | | 2 | | | 5 | | |
| | 57 | 64 | 72 | 57 | 76 | 98 | 57 | 81 | 158 |
| | IgG | PR2005+ OKT3 | OKT3 | IgG | PR2005+ OKT3 | OKT3 | IgG | PR2005+ OKT3 | OKT3 |

| | IL-10 | | | | | | | | |
|---|---|---|---|---|---|---|---|---|---|
| Time (hrs) Concentration (pg/ml) | 1 | | | 2 | | | 5 | | |
| | 694 | 882 | 3319 | 694 | 1961 | 11001 | 694 | 8614 | 20052 |
| | IgG | PR2005+ OKT3 | OKT3 | IgG | PR2005+ OKT3 | OKT3 | IgG | PR2005+ OKT3 | OKT3 |

| | TNF-α | | | | | | | | |
|---|---|---|---|---|---|---|---|---|---|
| Time (hrs) Concentration (pg/ml) | 1 | | | 2 | | | 5 | | |
| | 51 | 438 | 1409 | 51 | 412 | 1127 | 51 | 110 | 655 |
| | IgG | PR2005+ OKT3 | OKT3 | IgG | PR2005+ OKT3 | OKT3 | IgG | PR2005+ OKT3 | OKT3 |

Conclusion: the test results show that PR2005 can inhibit the OKT3-induced immune storm in an animal model.

## Claims

1. Use of progestogen in the manufacture of a medicament for inhibiting a cytokine storm syndrome.

2. The use according to claim 1, wherein the progestogen is hydroxyprogesterone caproate.

3. The use according to claim 1 or 2, wherein the cytokine storm syndrome is induced by various virus infections, macromolecular antibody therapy, organ transplantation or chimeric antigen receptor T cell (CAR-T) therapy.

4. The use according to claim 3, wherein the cytokine storm syndrome is induced by coronavirus, influenza virus and other virus infections.

5. The use according to claim 4, wherein the cytokine storm syndrome is induced by novel coronavirus infection.

6. A pharmaceutical composition for inhibiting a cytokine storm syndrome, comprising progestogen.

7. The pharmaceutical composition according to claim 6, wherein the progestogen is hydroxyprogesterone caproate.

8. The pharmaceutical composition according to claim 6 or 7, wherein the cytokine storm syndrome is induced by various virus infections, macromolecular antibody therapy, organ transplantation or chimeric antigen receptor T cell (CAR-T) therapy.

9. The pharmaceutical composition according to claim 8, wherein the cytokine storm syndrome is induced by coronavirus infection.

10. The pharmaceutical composition according to claim 9, wherein the cytokine storm syndrome is induced by novel coronavirus infection.

11. A method for treating a coronavirus-infected disease, comprising administering progestogen.

12. The method according to claim 11, wherein the progestogen is hydroxyprogesterone caproate.

13. The method according to claim 11 or 12, wherein the coronavirus is a novel coronavirus.

14. The method according to any one of claims 11-13, wherein the coronavirus-infected disease is novel coronavirus pneumonia.

15. The method according to any one of claims 11-14, comprising administering the progestogen to inhibit a cytokine storm syndrome in the coronavirus-infected disease.
